# EUROPEAN PATENT SPECIFICATION

(11) **EP 0 585 981 B1**
(45) Date of publication and mention of the grant of the patent: **18.06.1997**
(21) Application number: 93200750.3
(22) Date of filing: 15.03.1993
(51) Int. Cl.: A61K 7/025

(54) **Lipcolor composition**
Zusammensetzung zur Lippenpflege
Composition pour le traitement de lèvres

(30) Priority: 06.08.1992 JP 233144/92
(43) Date of publication of application: 09.03.1994
(73) Proprietor: KANEBO LTD., Tokyo (JP)
(72) Inventor: Sato, Nobumasa, Odawara-shi, Kanagawa (JP)
(74) Representative: Smulders, Theodorus A.H.J., Ir.

(56) References cited:
- EP-A- 0 549 267
- FR-A- 2 604 625
- US-A- 4 795 631
- US-A- 5 108 737
- PATENT ABSTRACTS OF JAPAN vol. 13, no. 308 (C-617) & JP-A-01 096 111 (KANEBO LTD)
- PATENT ABSTRACTS OF JAPAN vol. 12, no. 491 (C-554) & JP-A-63 201 109 (KOBAYASHI KOOC K. K.)

## Description

### FIELD OF THE INVENTION

The present invention relates to a lipcolor composition, particularly a lipcolor composition which less yields a dry feeling and an uneasy feeling with the passage of time, and whose color comes off less in friction with foods, drinks and so on.

### BACKGROUND OF THE INVENTION

Lipcolors intrinsically function to make up lips, make a complexion better and make an expression brighter. Now, the increasing number of women have jobs and are working actively. Lipcolors play a very important role in makeup effects, compared to other makeup cosmetics, as a lipcolor alone can produce a bright expression even without a makeup base or other cosmetics. Accordingly, many working women tend to just arrange hair styles, pencil eyebrows and put on lipcolor. Then, busy women want to save time of fixing their lipcolor and are interested in durability of makeup effects more than ever. However, lipcolor compositions without an oil component and a coloring component were hardly conceivable from a point of view of satisfactory makeup effects and physical feelings. Accordingly, only limited means were allowed to enhance durability of the makeup effects. That is, most popular ways are to blend a large amount of a highly sticky or solid oil component or a coloring agent, or to blend a coloring substance having a dyeing property. There is a little elaborate method where a volatile component is blended and, upon its volatization, there remain only a powdery component, a solid oil component and a highly sticky oil component. Unfortunately, lipsticks produced in these methods are not durable to friction with foods, drinks and so on, loose their comfortable feeling of use and tend to discolor and cause a dry feeling with the passage of time. Thus, intrinsic functions of lipsticks are lost easily.

The present inventor proposed oily makeup cosmetics comprising divalent or trivalent metal or its salt or hydroxide, a water-soluble salt of alginic acid and an oil component (Japanese Patent Application Laid-Open Hei-1-96111). The oil components described there are olive oil, castor oil, jojoba oil, lanoline, vaseline, squalane, liquid paraffin, octyl dodecanol, glyceryl tri(caprylate/caprate) and silicone oil. The oily makeup cosmetics include a lipstick which comprises D.& C. Red No. 7 (C.I. No. 15850) (see the present specification, Comparison Example 4) and Aluminium lake of F.D.& C. Yellow No. 5 (C.I. No. 19140); candelilla wax, carnauba wax, vaseline, lanoline, octyl dodecanol and castor oil; calcium pantothenate, calcium lactate or calcium sulfate; and sodium alginate.

### SUMMARY OF THE INVENTION

A purpose of the invention is to provide a lipcolor composition which less yields a dry feeling and an uneasy feeling with the passage of time and whose color comes off less in friction with foods, drinks and so on.

The present invention is a lipcolor composition comprising (a) at least one metal compound selected from the group consisting of salts of divalent and trivalent metals and hydroxides of divalent and trivalent metals, and (b) a water-soluble salt of alginic acid, characterized in that the composition further comprises (c) at least one red or orange lake color with aluminium, barium or zirconium derived from a color having a COONa or COOK group in its structure, selected from the group consisting of aluminium, barium or zirconium lakes of D.&C. Red No. 28; Rose bengal, D.&C. Red No. 22; D.&C. Orange No. 11; Eosine YSK; Phloxine BK; or Rose bengal K, and (d) an oil component having appearance of liquid at a temperature of 20°C.

### PREFERRED EMBODIMENTS OF THE INVENTION

The salts of divalent or trivalent metals used in the invention are known. Examples of the salts include calcium chloride, calcium carbonate, calcium lactate, calcium pantothenate, calcium sulfate, calcium citrate, calcium glycerophosphate, calcium gluconate, calcium hydrogenpyrophosphate, calcium biphosphate, calcium silicate, calcium stearate, calcium mesotartrate, calcium laurate, calcium acetate, aluminium sulfate, aluminium chloride, potassium alum, ferrous sulfate, ferric chloride, ferrous or ferric citrate, ferrous or ferric lactate, ferrous pyrophosphate, and sodium ferrous citrate. Water-insoluble salts such as barium sulfate are not preferred as they hardly react on lips. Hardly soluble salts such as calcium sulfate may be used. Preferred are calcium lactate, calcium pantothenate and calcium sulfate. The hydroxides of divalent or trivalent metals include calcium hydroxide, aluminium hydroxide and ferrous hydroxide.

The water-soluble salt of alginic acid used in the invention is known and includes sodium alginate, potassium alginate and ammonium alginate. The molecular weight of alginic acid is preferably 50,000 to 200,000, particularly 100,000 to 200,000, but not limited to these. The use of the higher molecular weight gives better results in the invention.

The red or orange colors having a COONa or COOK group in their structure are known. Those having a COONa group include D.& C. Red No. 28 (C.I. No. 45410); Rose bengal (C.I. No. 45440); D.& C. Red No. 22 (C.I. No. 45380); and D.& C. Orange No. 11 (C.I. No. 45425). Those having a COOK group include Eosine YSK (C.I. No. 45380); Phloxine BK (C.I. No. 45410); and Rose bengal K (C.I. No. 45440). The lakes of the colors used in the invention are lakes of the aforesaid colors with aluminium, barium, zirconium or the like.

The oil component which has appearance of liquid at a temperature of 20 °C includes monohydric alcohols having at least 16 carbon atoms; esters of monohydric alcohols having at least 3 carbon atoms or cholesterols with monobasic aliphatic carboxylic acids having at least 8 carbon atoms, adipic acid, oxystearic acid, succinic acid or malic acid; and esters of ethylene glycol, propylene glycol, neopentyl glycol, glycerin, diglycerin, trimethylol propane or pentaerythrytol with monobasic aliphatic carboxylic acids having at least 7 carbon atoms.

The monohydric alcohols having at least 16 carbon atoms, which have appearance of liquid at 20 °C and are used in the invention, are known and include isosteary alcohol, 2-octyl dodecanol, oleyl alcohol, 2-hexyl decanol and jojoba alcohol.

The ester of monohydric alcohols having at least 3 carbon atoms or cholesterols with monobasic aliphatic carboxylic acids having at least 8 carbon atoms, adipic acid, oxystearic acid, succinic acid or malic acid, which have appearance of liquid at 20 °C and are used in the invention, are known and include diisostearyl adipate, dioctyl adipate, isopropyl isostearate, cholesteryl isostearate, isononyl isononanoate, octyldodecyl erucate (ester of erucic acid), octyl oxystearate, cetyl octanoate, octyldodecyl oleate, oleyl oleate, dioctyl succinate, isocetyl stearate, isopropyl palmitate, octyldodecyl myristate, myristyl myristate, diisostearyl malate, decyl oleate, and octyldodecyl ricinoleate.

The esters of ethylene glycol, propylene glycol, neopentyl glycol, glycerin, diglycerin, trimethylol propane or pentaerythrytol with monobasic aliphatic carboxylic acids having at least 7 carbon atoms, which have appearance of liquid at 20 °C and are used in the invention, are known and include ethylene glycol octanoate, ethyleneglycol palmitate, propylene glycol isostearate, propylene glycol ricinoleate, propylene glycol di(caprylate/caprate), propylene glycol dicaprylate, propylene glycol dimyristate, propylene glycol dicaprate, neopentyl glycol dioctanoate, neopentyl glycol dicaprate, glyceryl triisostearate, glyceryl diisoastearate, glyceryl trioctanoate, glyceryl tri (caprylate/caprate), glyceryl ricinoleate, diglyceryl isostearate, diglyceryl diisostearate, diglyceryl triisostearate, trimethylol propane trioctanoate, trimethylol propane triisostearate, and pentaerythritol tetraoctanoate.

The lipcolor composition according to the invention may be in a form of solid, liquid or powder.

The lipcolor in the form of solid preferably contains (a) 0.1 to 5 parts by weight, particularly 0.5 to 3 parts by weight, of the salt or hydroxide of divalent or trivalent metal, (b) 0.1 to 5 parts by weight, particularly 0.5 to 3 parts by weight, of the water-soluble salt of alginic acid, (c) 3 to 20 parts by weight, particularly 3 to 10 parts by weight, of the red or orange lake color having a COONa or COOK group in its structure, and (d) 15 to 45 parts by weight, particularly 15 to 30 parts by weight, of the oil component having appearance of liquid at a temperature of 20 °C.

The lipcolor in the form of liquid preferably contains (a) 0.5 to 10 parts by weight, particularly 1 to 5 parts by weight, of the salt or hydroxide of divalent or trivalent metal, (b) 0.5 10 parts by weight, particularly 1 to 5 parts by weight, of the water-soluble salt of alginic acid, (c) 1 to 15 parts by weight, particularly 3 to 10 parts by weight, of the red or orange lake color having a COONa or COOK group in its structure, and (d) 15 to 98 parts by weight, particularly 20 to 50 parts by weight, of the oil component having appearance of liquid at 20 °C.

The lipcolor in the form of powder preferably contains (a) 1 to 20 parts by weight, particularly 2 to 10 parts by weight, of the salt or hydroxide of divalent or trivalent metal, (b) 1 to 20 parts by weight, particularly 2 to 10 parts by weight, of the water-soluble salt of alginic acid, (c) 4 to 40 parts by weight, particularly 10 to 30 parts by weight, of the red or orange lake color having a COONa or COOK group in its structure, and (d) 15 to 35 parts by weight, particularly 15 to 30 parts by weight, of the oil component having appearance of liquid at 20 °C.

The solid lipcolor of the invention may further contain conventional components, such as solid oil components, e.g., paraffins, ceresin and beeswax, pasty oil components, e.g., vaseline, lanolin and heavy liquid isoparaffins, liquid oil components, e.g., liquid paraffin and squalane, other organic coloring matters, inorganic pigments such as titanium oxide, antioxidants, preservatives and perfumes in addition to the aforesaid essential components, as long as these do not interfere with the pursuance of the purpose of the invention.

The liquid lipcolor of the invention may further contain conventional components, such as solid oil components, e.g., paraffins, ceresin and beeswax, oil-soluble thickening agents such as silicic anhydride, succrose esters of aliphatic acids and 12-hydroxy stearic acid, pasty oil components, e.g., vaseline, lanolin and heavy liquid isoparaffins, liquid oil components, e.g., liquid paraffin, squalane and silicone oils, other organic coloring matters, inorganic pigments such as titanium oxide, antioxidants, preservatives and perfumes in addition to the aforesaid essential components, as long as these do not interfere with the pursuance of the purpose of the invention.

The powder lipcolor of the invention may further contain conventional components, such as pigments, e.g., talc, mica, mica titanium, mica titanium treated with iron oxide, nylon powder and silk powder, solid oil components, e.g., paraffins, ceresin and beeswax, pasty oil components, e.g., vaseline, lanolin and heavy liquid isoparaffins, liquid oil components, e.g., liquid paraffin, squalane, dimethyl polysiloxane and methyl phenyl polysiloxane, other organic coloring matters, inorganic pigments such as titanium oxide, antioxidants, preservatives and perfumes in addition to the aforesaid essential components, as long as these do not interfere with pursuance of the purpose of the invention.

The present invention will be further explained in reference to the following Examples.

### EXAMPLES

In the Examples, the following evaluation item and method were adopted.

130 Female subjects were randomly grouped into 13 groups with each 10 members. Each group was provided with one out of 10 samples according to the invention and 3 samples for comparison. Each member puts her given lipcolor and, 2 hours later, ate Chinese foods which have a largest effect of removing lipcolor, and evaluated the lipcolor for its coming off.

### Examples 1 to 10 and Comparative Examples 1 to 3

Solid lipcolors were prepared in a conventional manner using the components shown in Table 1 in the amounts shown in percent by weight. That is, components 1 through 14 were mixed and dissolved uniformly at 90 °C, to which components 15 through 21 were added, kneaded and heat melted at 80 °C. After deaeration, the mixture was poured in a mold and cooled to be shaped, and put into a receptacle to obtain a product lipcolor.

The results of the evaluation are as shown in Table 2, where the number of the people who answerd that the lipcolor did not come off is indicated. Nobody had a dry or uneasy feeling 2 hours after the lipcolor was put in the Examples or the Comparion Examples, either.

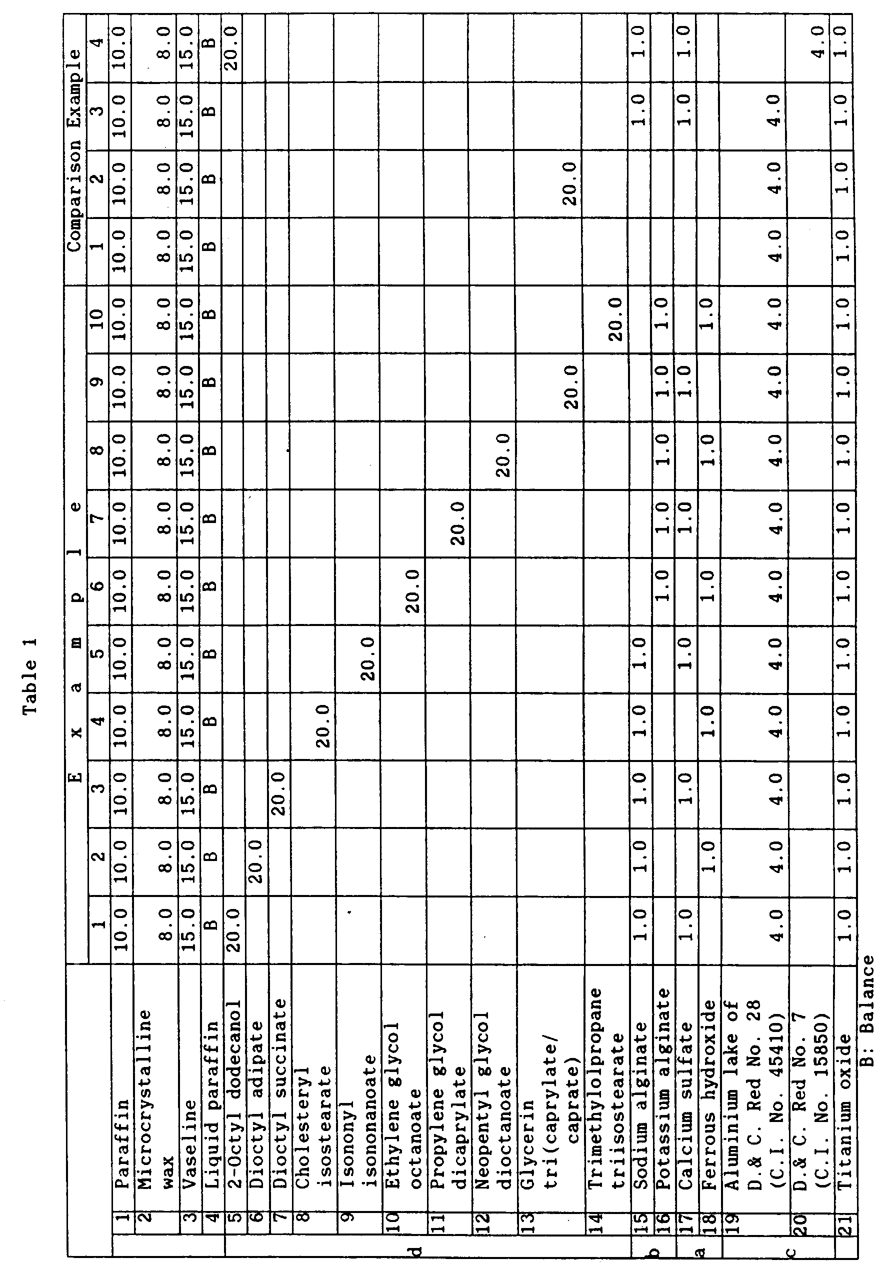

**Table 2**

| | Example | | | | | | | | | | Comparison | | | |
|---|---|---|---|---|---|---|---|---|---|---|---|---|---|---|
| | 1 | 2 | 3 | 4 | 5 | 6 | 7 | 8 | 9 | 10 | 1 | 2 | 3 | 4 |
| No coming-off | 8 | 8 | 8 | 9 | 8 | 8 | 10 | 8 | 10 | 8 | 0 | 5 | 3 | 0 |

### Example 12

Liquid lipcolor with the following composition was prepared in a conventional manner, and subjected to a test as in Example 1. The results were good similarly.

| | | |
|---|---|---|
| (d) | Diisostearyl malate | 10.0 wt.% |
| (d) | Pentaerythritol tetraoctanoate | 16.0 wt.% |
| | Dimethyl polysilaxane | 3.0 wt.% |
| | Silicic anhydride | 2.5 wt.% |
| | Liquid paraffin | 58.1 wt.% |
| (b) | Sodium alginate | 2.0 wt.% |
| (a) | Calcium pantothenate | 2.0 wt.% |
| (c) | Aluminium lake of Eosine YSK (C.I. No. 45380) | 4.5 wt.% |
| | Aluminium lake of F.D.& C. Blue No. 1 (C.I. No. 42090) | 0.5 wt.% |
| | Yellow oxide | 1.4 wt.% |

### Example 13

Powder lipcolor with the following composition was prepared in a conventional manner, and subjected to a test as in Example 1. The results were good similarly.

| | | |
|---|---|---|
| (d) | Diglyceryl isostearate | 10.0 wt.% |
| (d) | Octyl oxystearate | 17.0 wt.% |
| (b) | Potassium alginate | 8.0 wt.% |
| (a) | Aluminium hydroxide | 5.0 wt.% |
| (c) | Aluminim lake of Phloxine BK (C.I. No. 45410) | 5.0 wt.% |
| (c) | Barium lake of D.& C. Red No. 28 (C.I. No. 45410) | 12.0 wt.% |
| | Aluminium lake of F.D.& C. Blue No. 1 (C.I. No. 42090) | 0.5 wt.% |
| | Barium sulfate | 5.0 wt.% |
| | Mica | 17.5 wt.% |
| | Mica titanium | 20.0 wt.% |

## Claims

1. A lipcolor composition comprising (a) at least one metal compound selected from the group consisting of salts of divalent and trivalent metals and hydroxides of divalent and trivalent metals, and (b) a water-soluble salt of alginic acid, characterized in that the composition further comprises (c) at least one red or orange lake color with aluminium, barium or zirconium derived from a color having a COONa or COOK group in its structure, selected from the group consisting of aluminium, barium or zirconium lakes of D.&C. Red No. 28 (C.I. No. 45410); Rose bengal (C.I. No. 45440), D.&C. Red No. 22 (C.I. No. 45380); D.&C. Orange No. 11 (C.I. No. 45425); Eosine YSK (C.I. No. 45380); Phloxine BK (C.I. No. 45410); or Rose bengal K (C.I. No. 45440), and (d) an oil component having appearance of liquid at a temperature of 20°C.

2. The lipcolor composition as claimed in claim 1, wherein the oil component which has appearance of liquid at a temperature of 20°C is selected from the group consisting of monohydric alcohols having at least 16 carbon atoms; esters of monohydric alcohols having at least 3 carbon atoms or cholesterols with monobasic aliphatic carboxylic acids having at leat 8 carbon atoms, adipic acid, oxystearic acid, succinic acid or malic acid; and esters of ethylene glycol, propylene glycol, neopentyl glycol, glycerin, diglycerin, trimethylol propane or pentaerylthrytol with monobasic aliphatic carboxylic acids having at least 7 carbon atoms.

3. The lipcolor composition as claimed in claim 2, wherein the monohydric alcohols having at least 16 carbon atoms are isostearyl alcohol, 2-octyl dodecanol, oleyl alcohol, 2-hexyl decanol and jojoba alcohol; the ester of monohydric alcohols having at least 3 carbon atoms or cholesterols with monobasic aliphatic carboxylic acids having at least 8 carbon atoms, adipic acid, oxystearic acid, succinic acid or malic acid are diisostearyl adipate, dioctyl adipate, isopropyl isostearate, cholesteryl isostearate, isononyl isononanoate, octyldodecyl erucate (ester of erucic acid), octyl oxystearate, cetyl octanoate, octyldodecyl oleate, oleyl oleate, dioctyl succinate, isocetyl stearate, isopropyl palmitate, octyldodecyl myristate, myristyl myristate, diisostearyl malate, decyl oleate, and octyldedecyl ricinoleate; and the esters of ethylene glycol, propylene glycol, neopentyl glycol, glycerin, diglycerin, trimethylol propane or pentaerylthrytol with monobasic aliphatic carboxylic acids having at least 7 carbon atoms are ethylene glycol octanoate, ethyleneglycol palmitate, propylene glycol isostearate, propylene glycol ricinoleate, propylene glycol di(caprylate/caprate), propylene glycol dicaprylate, propylene glycol dimyristate, propylene glycol dicaprate, neopentyl glycol dioctanoate, neopentyl glycol dicaprate, glyceryl triisostearate, glyceryl diisoastearate, glyceryl trioctanoate, glyceryl tri(caprylate/caprate), glyceryl ricinoleate, diglyceryl isostearate, diglyceryl diisostearate, diglyceryl triisostearate, trimethylol propane trioctanoate, trimethylol propane triisostearate, and pentaerythritol tetraoctanoate.

4. The lipcolor composition as claimed in claim 1, wherein the lipcolor is in a form of solid and contains (a) 0.1 to 5 parts by weight of the salt or hydroxide of divalent or trivalent metal, (b), 0.1 to 5 parts by weight of the water-soluble salt of alginic acid, (c) 3 to 20 parts by weight of the red or orange lake color having a COONa or COOK group in its structure, and (d) 15 to 45 parts by weight of the oil component having appearance of liquid at a temperature of 20°C.

5. The lipcolor composition as claimed in claim 1, wherein the lipcolor is in a form of liquid and contains (a) 0.5 to 10 parts by weight of the salt or hydroxide of divalent or trivalent metal, (b) 0.5 to 10 parts by weight of the water-soluble salt of alginic acid, (c) 1 to 15 parts by weight of the red or orange lake color having a COONa or COOK group in its structure, and (d) 15 to 98 parts by weight of the oil component having appearance of liquid at a temperature of 20°C.

6. The lipcolor composition as claimed in claim 1, wherein the lipcolor is in a form of powder and contains (a) 1 to 20 parts by weight of the salt or hydroxide of divalent or trivalent metal, (b) 1 to 20 parts by weight of the water-soluble salt of alginic acid, (c) 4 to 40 parts by weight of the red or orange lake color having a COONa or COOK group in its structure, and (d) 15 to 35 parts by weight of the oil component having appearance of liquid at a temperature of 20°C.

7. The lipcolor composition as claimed in claim 1, wherein the water-soluble salt of alginic acid is sodium alginate, potassium alginate or ammonium alginate and a molecular weight of the alginic acid is 100,000 to 200,000.

8. The lipcolor composition as claimed in claim 1, wherein the salts of divalent or trivalent metals are calcium chloride, calcium carbonate, calcium lactate, calcium pantothenate, calcium sulfate, calcium citrate, calcium glycerophosphate, calcium gluconate, calcium hydrogenpyrophosphate, calcium biphosphate, calcium silicate, calcium stearate, calcium mesotartrate, calcium laurate, calcium acetate, aluminium sulfate, aluminium chloride, potassium alum, ferrous sulfate, ferric chloride, ferrous or ferric citrate, ferrous or ferric lactate, ferrous pyrophosphate and sodium ferrous citrate; and the hydroxides of divalent or trivalent metals are calcium hydroxide, aluminium hydroxide and ferrous hydroxide.

## Patentansprüche

1. Zusammensetzung zur Lippenpflege, umfassend (a) wenigstens eine Metallverbindung, ausgewählt aus der Gruppe bestehend aus Salzen von zweiwertigen und dreiwertigen Metallen und Hydroxiden von zweiwertigen und dreiwertigen Metallen, und (b) einem wasserlöslichen Salz von Alginsäure, **dadurch gekennzeichnet**, daß die Zusammensetzung weiterhin umfaßt (c) wenigstens eine rote oder orange Lackfarbe mit Aluminium, Barium oder Zirkonium, die sich von einer Farbe ableitet mit einer COONa- oder COOK-Gruppe in ihrem Aufbau, ausgewählt aus der Gruppe bestehend aus Aluminium-, Barium- oder Zirkoniumlackfarben von D.&C. Red Nr. 28 (C.I. Nr. 45410); Rose bengal (C.I. Nr. 45440), D.&C. Red Nr. 22 (C.I. Nr. 45380); D.&C. Orange Nr. 11 (C.I. Nr. 45425); Eosine YSK (C.I. Nr. 45380); Phloxine BK (C.I. Nr. 45410); oder Rose bengal K (C.I. Nr. 45440), und (d) eine Ölkomponente, die bei einer Temperatur von 20°C ein flüssiges Aussehen hat.

2. Zusammensetzung zur Lippenpflege gemäß Anspruch 1, in welcher die Ölkomponente, die das Aussehen einer Flüssigkeit bei einer Temperatur von 20°C hat, ausgewählt ist aus der Gruppe bestehend aus einwertigen Alkoholen mit wenigstens 16 Kohlenstoffatomen; Estern von einwertigen Alkoholen mit wenigstens 3 Kohlenstoffatomen oder Cholesterolen mit einwertigen aliphatischen Carbonsäuren mit wenigstens 8 Kohlenstoffatomen, Adipinsäure, Oxystearinsäure, Bernsteinsäure oder Apfelsäure; und Estern von Ethylenglykol, Propylenglykol, Neopentylglykol, Glycerin, Diglycerin, Trimethylolpropan oder Pentaerythritol mit einbasigen aliphatischen Carbonsäuren mit wenigstens 7 Kohlenstoffatomen.

3. Zusammensetzung zur Lippenpflege gemäß Anspruch 2, in welcher die einwertigen Alkohole mit wenigstens 16 Kohlenstoffatomen Isostearylalkohol, 2-Octyldodecanol, Oleylalkohol, 2-Hexyldecanol und Jojobaalkohol sind; die Ester von einwertigen Alkoholen mit wenigstens 3 Kohlenstoffatomen oder Cholesterolen mit einbasischen aliphatischen Carbonsäuren mit wenigstens 8 Kohlenstoffatomen, Adipinsäure, Oxystearinsäure, Bernsteinsäure oder Apfelsäure sind Diisostearyladipat, Dioctyladipat, Isopropylisostearat, Cholesterylisostearat, Isononylisononanoat, Octyldodecylerucat (Ester von Erucinsäure), Octyloxystearat, Cetyloctanoat, Octyldodecyloleat, Oleyloleat, Dioctylsuccinat, Isocetylstearat, Isopropylpalmitat, Octyldodecylmyristat, Myristylmyristate, Diidostearylmalat, Decyloleat und Octyldedecylricinoleat; und die Ester von Ethylenglykol, Propylenglykol, Neopentylglykol, Glycerin, Diglycerin, Trimethylolpropan oder Pentaerylthrytol mit einwertigen aliphatischen Carbonsäuren mit wenigstens 7 Kohlenstoffatomen sind Ethylenglykoloctanoat, Ethylenglykolpalmitat, Propylenglykolisostearat, Propylenglykolricinoleat, Propylenglykoldi (caprylat/caprat), Propylenglykoldicaprylat, Propylenglykoldimyristat, Propylenglykoldicaprat, Neopentylglykoldioctanoat, Neopentylglykoldicaprat, Glyceryltriisostearat, Glyceryldiisostearat, Glyceryltrioctanoat, Glyceryltri(caprylat/caprat), Glycerylricinoleat, Diglycerylisostearat, Diglyceryldiisostearat, Digylceryltriisostearat, Trimethylolpropantrioctanoat, Trimethylolpropantriisostearat und Pentaerythritoltetraoctanoat.

4. Zusammensetzung zur Lippenpflege gemäß Anspruch 1, in welcher die Lippenfarbe in Form eines Feststoffes vorliegt und (a) 0,1 bis 5 Gewichtsteile des Salzes oder Hydroxids eines zweiwertigen oder dreiwertigen Metalls, (b) 0,1 bis 5 Gewichtsteile des wasserlöslichen Salzes von Alginsäure, (c) 3 bis 20 Gewichtsteile der roten oder orangen Lackfarbe mit einer COONa- oder COOK-Gruppe in ihrem Aufbau, und (d) 15 bis 45 Gewichtsteile der Ölkomponente, die bei 20°C das Aussehen einer Flüssigkeit hat, enthält.

5. Zusammensetzung zur Lippenpflege gemäß Anspruch 1, in welcher die Lippenfarbe in Form einer Flüssigkeit vorliegt und (a) 0,5 bis 10 Gewichtsteile des Salzes oder Hydroxids eines zweiwertigen oder dreiwertigen Metalls, (b) 0,5 bis 10 Gewichtsteile des wasserlöslichen Salzes von Alginsäure, (c) 1 bis 15 Gewichtsteile der roten oder orangen Lackfarbe mit einer COONa- oder COOK-Gruppe in ihrem Aufbau, und (d) 15 bis 98 Gewichtsteile der Ölkomponente, die bei 20°C das Aussehen einer Flüssigkeit hat, enthält.

6. Zusammensetzung zur Lippenpflege gemäß Anspruch 1, in welcher die Lippenfarbe in Form eines Pulvers vorliegt und (a) 1 bis 20 Gewichtsteile des Salzes oder Hydroxids eines zweiwertigen oder dreiwertigen Metalls, (b) 1 bis 20 Gewichtsteile des wasserlöslichen Salzes von Alginsäure, (c) 4 bis 40 Gewichtsteile der roten oder orangen Lackfarbe mit einer COONa- oder COOK-Gruppe in ihrem Aufbau, und (d) 15 bis 35 Gewichtsteile der Ölkomponente, die bei 20°C das Aussehen einer Flüssigkeit hat, enthält.

7. Zusammensetzung zur Lippenpflege gemäß Anspruch 1, in welcher das wasserlösliche Salz der Alginsäure Natriumalginat, Kaliumalginat oder Ammoniumalginat ist und das Molekulargewicht der Alginsäure 100.000 bis 200.000 ist.

8. Zusammensetzung zur Lippenpflege gemäß Anspruch 1, in welcher die Salze von zweiwertigen oder dreiwertigen Metallen Calciumchlorid, Calciumcarbonat, Calciumlactat, Calciumpanthothenat, Calciumsulfat, Calciumcitrat, Calciumglycerophosphat, Calciumgluconat, Calciumhydrogenpyrophosphat, Calciumbiphosphat, Calciumsilicat, Calciumstearat, Calciummesotartrat, Calciumlaurat, Calciumacetat, Aluminiumsulfat, Aluminiumchlorid, Kaliumalaun, Eisen(II)sulfat, Eisen(III)chlorid, Eisen(II)- oder Eisen(III)citrat, Eisen(II)- oder Eisen(III)lactat, Eisen(II)pyrrophosphat und Natriumeisen(II)citrat sind; und die Hydroxide von zweiwertigen oder dreiwertigen Metallen Calciumhydroxid, Aluminiumhydroxid und Eisen(II)hydroxid sind.

## Revendications

1. Composition pour la coloration des lèvres comprenant (a) au moins un composé métallique choisi dans le groupe constitué par les sels de métaux divalents et trivalents et les hydroxydes de métaux divalents et trivalent, et (b) un sel soluble dans l'eau d'acide alginique, caractérisée par le fait que la composition contient en outre (c) au moins un colorant de laque rouge ou orange d'aluminium, de barium ou de zirconium, dérivé d'un colorant ayant, dans sa structure, un groupe COONa ou COOK, choisi dans le groupe constitué par les laques d'aluminium, de barium ou de zirconium de D & C Red No. 28 (C.I. No. 45410), Rose bengale (C.I. No. 45440), D & C Red No. 22 (C.I. No. 45380); D & C Orange No. 11 (C.I. No. 45425); Eosine YSK (C.I. No. 45380), Phloxine BK (C.I. No. 45410), ou Rose bengale K (C.I. No 45440) et (d) un composant huileux ayant l'apparence d'un liquide à la température de 20° C.

2. Composition pour la coloration des lèvres telle que revendiquée selon revendication 1, dans laquelle le composé huileux qui a l'apparence d'un liquide à la température de 20° C est choisi dans le groupe constitué par les alcools monohydriques ayant au moins 16 atomes de carbone, les esters d'alcools monohydriques ayant au moins 3 atomes de carbone ou de cholestérols avec des acides carboxyliques monobasiques aliphatiques ayant au moins 8 atomes de carbone, l'acide adipique, l'acide oxystéarique, l'acide succinique ou l'acide malique, et les esters d'éthylène glycol, de propylène glycol, de néopentyle glycol, de glycérine, de diglycérine, de triméthylol propane ou de pentaérylthritol avec des acides carboxyliques monobasiques aliphatiques ayant au moins 7 atomes de carbone.

3. Composition pour la coloration des lèvres telle que revendiquée selon la revendication 2 dans laquelle les alcools monohydriques ayant au moins 16 atomes de carbone sont l'alcool isostéarylique, le 2-octyl dodécanol, l'alcool oléique, le 2-héxyl décanol et l'alcool de jojoba ; les esters d'alcools monohydriques ayant au moins 3 atomes de carbone ou de cholestérols avec des acides carboxyliques monobasiques aliphatiques avent au moins 8 atomes de carbone, l'acide adipique, l'acide oxystéarique, l'acide succinique ou l'acide malique sont choisis parmi l'adipate de diisostéaryl, l'adipate de dioctyle, l'isostérate d'isopropyl, l'isostérate de cholestéryle, l'isononanoate d'isononyle, l'érucate d'octyldodécyle (ester de l'acide érucique), l'oxystéarate d'octyle, l'octanoate de cétyle, l'oléate d'octyldodécyle, l'oléate d'oléyle, le succinate de dioctyle, le stérate d'isocétyle, le palmitate d'isopropyle, le myristate d'octyldodécyle, le myristate de myristyle, le malate de diisostéaryle, l'oléate de décyle et le ricinoléate d'octyldodécyle ; et les esters d'éthylène glycol, de propylène glycol, de néopentyle glycol, de glycérine, de diglycérine, de triméthylol propane ou de pentaérylthritol avec des acides carboxyliques monobasiques aliphatiques ayant au moins 7 atomes de carbone sont choisis parmi l'octanoate d'éthylène glycol, le palmitate d'éthylène glycol, l'isostéarate de propylène glycol, le ricinoléate de propylène glycol, le di (caprylate/caprate) de propylène glycol, le dicaprylate de propylène glycol, le dimyristate de propylène glycol, le dicaprate de propylène glycol, le dioctanoate de néopentyle glycol, le dicaprate de néopentyle glycol, le triisostéarate de glycéryl, le diisostéarate de glycéryle, le trioctanoate de glycéryle, le tri (caprylate/caprate) de glycéryle, le ricinoléate de glycéryle, l'isostéarate de diglycéryle le diisostéarate de diglycéryle, le triisostéarate de diglycéryle, le trioctanoate de triméthylol propane, le triisostérate de triméthylol propane et le tétraoctanoate de pentaérythritol.

4. Composition pour la coloration des lèvres telle que revendiquée selon la revendication 1, dans laquelle celle-ci est sous forme solide et contient (a) 0,1 à 5 parties on poids du sel ou de l'hydroxyde d'un métal divalent ou trivalent, (b), 0,1 à 5 parties en poids d'un sel soluble dans l'eau d'acide alginique, (c) 3 à 20 parties en poids d'un colorant de laque rouge ou orange ayant, dans sa structure, un groupe COONa ou COOK, et (d) 15 à 45 parties en poids d'un composant huileux ayant l'apparence d'un liquide à la température de 20° C.

5. Composition pour la coloration des lèvres telle que revendiquée selon la revendication 1, dans laquelle celle-ci est sous forme liquide et contient (a) 0,5 à 10 parties en poids du sel ou de l'hydroxyde d'un métal divalent ou trivalent, (b) 0,5 à 10 parties en poids d'un sel soluble dans l'eau d'acide alginique, (c) 1 à 15 parties en poids d'un colorant de laque rouge ou orange ayant, dans sa structure, un groupe COONa ou COOK, et (d) 15 à 98 parties en poids d'un composant huileux ayant l'apparence d'un liquide a la température de 20° C.

6. Composition pour la coloration des lèvres telle que revendiquée selon la revendication 1, dans laquelle celle-ci est sous forme d'une poudre et contient (a) 1 à 20 parties en poids du sel ou de l'hydroxyde d'un métal divalent ou trivalent, (b) 1 à 20 parties en poids d'un sel soluble dans l'eau d'acidealginique, (c) 4 à 40 parties en poids d'un colorant de laque rouge ou orange ayant, dans sa structure, un groupe COONa ou COOK, et (d) 15 à 35 parties en poids d'un composant huileux ayant l'apparence d'un liquide à la température de 20° C.

7. Composition pour la coloration des lèvres telle que revendiquée selon la renvendication 1 dans laquelle le sel soluble dans l'eau de l'acide alginique est l'alginate de sodium, l'alginate de potassium ou l'alginate d'ammonium, l'acide alginique ayant un poids moléculaire de 100 000 à 200 000.

8. Composition pour la coloration des lèvres telle que revendiquée selon la revendication 1 dans laquelle les sels de métaux divalents ou trivalents sont le chlorure de calcium, le carbonate de calcium, le lactate de calcium, le pantothénate de calcium, le sulfate de calcium, le citrate de calcium, le glycérophosphate de calcium, le gluconate de calcium, l'hydrogénopyrophosphate de calcium, le biphosphate de calcium, le silicate de calcium, le stéarate de calcium, le mésotartrate de calcium, le laurate de calcium, l'acétate de calcium, le sulfate d'aluminium, le chlorure d'aluminium, l'alum de potassium, le sulfate ferreux, le chlorure ferrique, le citrate ferreux ou ferrique, le lactate ferreux ou ferrique, le pyrophosphate ferreux et le citrate ferro-sodique et les hydroxydes de métaux divalents ou trivalents sont l'hydroxyde de calcium, l'hydroxyde d'aluminium et l'hydroxyde ferreux .
